# EUROPEAN PATENT SPECIFICATION

(11) **EP 2 749 286 B1**
(45) Date of publication and mention of the grant of the patent: **01.03.2017**
(21) Application number: 11764669.5
(22) Date of filing: 26.08.2011
(51) Int. Cl.: A61K 31/5415, A61P 15/18, A61K 9/00

(54) **USE OF NON-STEROIDAL ANTI-INFLAMMATORY DRUGS MELOXICAM AND PIROXICAM, ADMINISTERED INTRAVAGINALLY, FOR INTERRUPTION OF A WOMAN'S OVULATION PROCESS**
VERWENDUNG DER NICHTSTEROIDALEN ENTZÜNDUNGSHEMMER MELOXICAM UND PIROXICAM IN INTRAVAGINALRINGEN ZUR UNTERBRECHUNG DES EISPRUNGS BEI FRAUEN
UTILISATION DES ANTI-INFLAMMATOIRES NON STEROIDIENS MELOXICAM ET PIROXICAM DANS ANNEAUX INTRAVAGINAUX POUR INTERROMPRE L'OVULATION CHEZ LES FEMMES

(43) Date of publication of application: 02.07.2014
(73) Proprietor: Universidad De Santiago De Chile, CP 9170022, Santiago (CL)
(72) Inventor: CARDENAS SANKAN, Hugo, Código Postal 7254758, Santiago (CL); CROXATTO AVONI, Horacio, Código Postal 7254758, Santiago (CL); RABAGLIATI CANESSA, Franco, Código Postal 7254758, Santiago (CL); ZAPATA RAMIREZ, Paula, Código Postal 7254758, Santiago (CL); GALVEZ PEREZ, Paula, Código Postal 7254758, Santiago (CL); ALTBIR DRULLINSKY, Dora, Código Postal 9170022, Santiago (CL); VELASQUEZ CUMPLIDO, Luis, Código Postal 7254758, Santiago (CL)
(74) Representative: Isern-Jara, Nuria
(86) International application number: PCT/CL2011/000049
(87) International publication number: WO 2013/029194

(56) References cited:
- US-A1- 2010 087 402
- US-B1- 6 544 546
- BENAGIANO GIUSEPPE ET AL: "Hormonal contraception: present and future.", DRUGS OF TODAY (BARCELONA, SPAIN : 1998) DEC 2008 LNKD- PUBMED:19198700, vol. 44, no. 12, December 2008 (2008-12), pages 905-923, XP002670299, ISSN: 1699-3993
- KERNS J ET AL: "Vaginal ring contraception", CONTRACEPTION, GERON-X, INC., LOS ALTOS, CA, US, vol. 83, no. 2, 1 February 2011 (2011-02-01), pages 107-115, XP027599238, ISSN: 0010-7824, DOI: 10.1016/J.CONTRACEPTION.2010.07.008 [retrieved on 2011-01-12]
- JESAM CRISTIÁN ET AL: "Suppression of follicular rupture with meloxicam, a cyclooxygenase-2 inhibitor: potential for emergency contraception.", HUMAN REPRODUCTION (OXFORD, ENGLAND) FEB 2010 LNKD- PUBMED:19933235, vol. 25, no. 2, February 2010 (2010-02), pages 368-373, XP002670300, ISSN: 1460-2350
- BATA M S ET AL: "Delay of ovulation by meloxicam in healthy cycling volunteers: A placebo-controlled, double-blind, crossover study", JOURNAL OF CLINICAL PHARMACOLOGY, vol. 46, no. 8, August 2006 (2006-08), pages 925-932, XP009156774, ISSN: 0091-2700
- SALHAB A S ET AL: "Further investigation on meloxicam contraceptivity in female rabbits: Luteinizing unruptured follicles, a microscopic evidence.", CONTRACEPTION, vol. 67, no. 6, June 2003 (2003-06), pages 485-489, XP002670301, ISSN: 0010-7824
- MASSAI M R ET AL: "Does meloxicam increase the incidence of anovulation induced by single administration of levonorgestrel in emergency contraception? A pilot study", HUMAN REPRODUCTION, OXFORD UNIVERSITY PRESS, GB, vol. 22, no. 2, 1 February 2007 (2007-02-01), pages 434-439, XP002605238, ISSN: 0268-1161, DOI: 10.1093/HUMREP/DE1369 [retrieved on 2006-09-15] cited in the application
- 'Climacteric Abstracts of Oral Presentations', [Online] vol. 14, no. S1, 01 June 2011, pages 57 - 97, XP008176703 DOI: 10.3109/13697137.2011.583733 ISSN: 1369-7137 CLIMACTERIC, PARTHENON PUBLISHING, CARNFORTH, GB Retrieved from the Internet: <URL:http://informahealthcare.com/doi/pdf/1 0.3109/13697137.2011.583733>

## Description

### SCOPE OF THE INVENTION

The invention relates to the field of reproductive medicine, specifically the area of fertility regulation. This patent proposes the use of nonsteroidal anti-inflammatory compounds, specifically meloxicam and piroxicam, released by rings made of polymeric vinyl monomers loaded with drugs and inserted in the vagina to inhibit ovulation.

The inhibition of ovulation by oral meloxicam has been demonstrated by one of the present inventors (Croxatto et al,. See below) and the contraceptive effects of sex steroids released into the vagina have also been widely demonstrated. These results led us to experiment the absorption of meloxicam administered intravaginally into the peripheral blood flow

There is evidence supporting the use of nonsteroidal anti-inflammatory family of Oxican such as meloxicam or piroxicam, released intravaginally to regulate fertility in women.

### OBJECTIVE OF THE INVENTION

This invention aims topical intravaginal release preparation of piroxicam or meloxicam to inhibit ovulation in women and has the following advantages:
1) A monthly device is inserted into the vagina, as opposed to the daily oral pills.
2) It does not require the administration of sexual steroids that interfere with the menstrual cycle or have other side effects.
3) Women would insert and remove the device without the use of medical assistance.
4) The nonsteroidal anti-inflammatory drugs and the vinyl polymers have a low cost, therefore you can have inexpensive devices for women and low-income families.
5) Meloxicam and other nonsteroidal anti-inflammatory medicines are sold over the counter in most countries.
6) Meloxicam already has FDA approval as a commonly used anti-inflammatory drug.
7) The initial burst of the drug should be avoided in the stomach and intestine associated with oral administration of meloxicam and other nonsteroidal anti-inflammatory drugs. These high levels of the drug increases the risk of epithelial injury and bleeding associated with nonsteroidal antiinflammatory drugs.

### STATE OF THE ART

Recently a report was issued on enhancing the anovulatory effect of levonorgestrel by the oral administration of meloxicam. In this study, administration of single doses of levonorgestrel (1.5 mg. orally) or levonorgestrel + meloxicam (15 mg.) inhibited follicular rupture by 50% and 70%, respectively. (Masai et al., 2007).

Levonorgestrel is a synthetic progestin that shows agonistic activity to progesterone by binding to its receptors and is currently used in various contraceptive methods (I.S. Fraser et al, Eds, Estrogens and Progestins in Clinical Practice, 1998,.. Churchill Livingstone, London) .

Meloxicam belongs to the family of nonsteroidal anti-inflammatory drugs Oxican, which exert their effect by inhibiting prostaglandin synthesis. Meloxicam was approved by the FDA for chronic or occasional pain associated with arthritis and other diseases (G. Hardman and LE Limbird Eds., Goodman & Gilman's The Pharmacological Basis of Therapeutics, 9th Ed, 1996)

Meloxicam inhibits the COX-2 limiting enzyme in the synthesis of inflammatory mediators known as prostaglandin, which in turn have an important role during ovulation, since they participate in the breakdown of the dominant follicle follicular wall (references in Masai et al, Human Reproduction Volume 22, pages 434-439, 2007,. JESAM et al, Human Reproduction Volume 25, pages 368-373, 2010).

Jesam et al. (2010) issued reports that daily doses of meloxicam 15 or 30 mg. administered orally for 5 days during the last phase of the follicular phase, inhibited ovulation menstrual cycles by 50% and 91% respectively, without producing changes in the pattern of sex hormones or the duration of the menstrual cycle. This finding was the first evidence of the effect of meloxicam in the specific inhibition of ovulation in controlled clinical trials.

The present invention enables the administration of meloxicam into the vagina in order to inhibit ovulation in women. It differs from the proposed Massai et al. (2007) and Jesam et al. (2010) which proposed the oral administration of meloxicam for the same purpose.

Patent applications do not exist for the intravaginal meloxicam with the purpose of inhibiting ovulation in women.

However, oral meloxicam and other oxicam family members have been proposed for alleviating the symptoms of arthritis. Meloxicam released through an intravaginal device can also be effective for these diseases.

Intravaginal rings of different polymer compositions have been proposed and used for hormone replacement therapy and fertility control. Have the advantage of releasing hormones for several weeks, unlike oral formulations.

Most hormones contraceptive based formulations employ a combination of a progestin with an estrogen. Progestins are molecules with similar properties to progesterone, while estrogen share some properties with estradiol. Progestins in a suitable dose inhibits ovulation and antagonize or block the effect of estrogen procarcinogenic. The estrogen in birth control formulas enhance the anti-ovulatory effect of progestins and regulate the pattern of vaginal bleeding associated with chronic elevation of progestins in the blood of some women.

There are several patents which disclose vaginal rings designed to release progestins and/or estrogens in the vagina:

U.S. Patent No. 4.596.576**.** Presents a two-compartment vaginal ring each containing a different active compound. However, to prevent dispersion and maintain a constant release ratio between the various active compounds over time, the compartments are separated by impermeable inert caps made of glass, silver and gold.

U.S. Patent No. 4.237.885**.** Presents a delivery system composed of silicone segments separated from each other by spacers, each loaded with a different molecule. The simplest device is a ring loaded with a single active ingredient suspended in silicone.

EP 876815 Presents an Intravaginal delivery system which has a ring shape and which delivers a progestin and an estrogen in a fixed ratio during prolonged periods of time.

EP 836473**.** Refers to a ring shaped device which contains a compartment with a copolymer center of ethylene-vinyl acetate without medication, covered with a layer of the same material charged with a steroid hormone and a non-medicated outer layer, also of the same material; a second compartment consisting of a copolymer center of ethylene-vinyl acetate loaded with a steroid hormone and an outer layer of the same material also contains optionally placebo compartments of a thermoplastic material separating compartments one and two. In a more preferred device, this invention relates to a vaginal ring with two compartments where the former is a crystalline etonogestrel and the second in a mixture of (sub)-saturated etonogestrel and ethinyl estradiol, both compartments are separated optionally one from the other by high density polyethylene placebo compartments.

WO 1995000199**.** Describes an intravaginal delivery system consisting of a flexible support means and a delivery medium containing the active agent, adhered to the support means. The support means consists of a central portion substantially open ring shaped, and the delivery medium consisting of at least one polymer body sleeve shaped which covers the central part as a belt along the central body portion.

WO 2005089723**.** Relates to a drug delivery system consisting of one or more compartments and in one progestogenic compound dissolved in a vinyl acetate copolymer of thermoplastic polyethylene. If the delivery system consists of one compartment, then the compartment comprises: (i) a core of thermoplastic polyethylene to the progestogenic compound vinyl acetate copolymer, for example a progestogenic compound dissolved in the copolymer vinyl acetate of polyethylene in a concentration less than the saturation level at 25 ° C and an estrogenic compound, and (ii) a permeable membrane of vinyl acetate copolymer of thermoplastic polyethylene for both compounds that covers the core. If the delivery system consists of more than one compartment, only one contains (iii) the progestogenic compound, for example a progestogenic compound dissolved in a core of thermoplastic polyethylene vinyl acetate copolymer with a concentration less than the saturation level at 25 ° C, and an estrogenic compound; and a permeable membrane of vinyl acetate copolymer of thermoplastic polyethylene for both compounds that cover the core.

EP 862396**.** Relates to a vaginal ring containing a body made from a polymeric primary material having at least one inner hollow channel that has an opening to the outside and that is adapted to receive a core containing the drug through the opening, and a core containing at least one drug to be administered intravaginally scattered in a secondary polymeric material disposed in the channel. The core is placed correctly in the vaginal ring before it is used primarily to avoid bursts of drug to tissues that may trigger side effects such as nausea or vomiting.

Vaginal rings exert their action over several weeks after insertion, which is easier than taking steroid pills daily at a particular schedule. These rings are inserted and removed easily when the woman wants and can be used according to your needs. Vaginal rings offer no protection or treatment against sexually transmitted diseases, but U.S. Patent 20100285097 discloses a device with both contraceptive and microbicidal activity.

US 20100285097**.** The invention is related to intravaginal system for controlled release of drospirenone and an estrogen. Optionally it can also consist of one or more therapeutically active substances or to improve health properties to provide and / or enhance the protection against bacterial or fungal product, while increasing protection against STDs substances. The delivery system comprises one or more compartments with a core and an overlying membrane. The core and the membrane essentially consist of either the same or different polymeric compositions, where at least one of the compartments contains drospirenone and at least one, which may be the same or different from that, contains an estrogen or a mixture of drospirenone and estrogen. The Outer membrane or at least one core contains the active therapeutic substance or to improve health. "Climacteric Abstracts of Oral Presentations",(ISSN:13697137)pages 66-67, with the title: "Meloxicam-releasing vaginal ring:A non hormonal contraceptive for women approaching the menopause" (2011-06-01) is a disclosure of the inventors of the present invention. It discloses the ability meloxicam (15-30 mg/day) to delay or prevent follicular rupture in normally cycling women, further indicating the potential utility of COX-2 inhibitors to abolish ovulation and prevent pregnancy. It outlines that the incidence of gastric disturbances associated with chronic use of COX-2 inhibitors could be minimized using a galenic formulation that delivers the drug in the vagina. It further indicates that a vaginal ring releasing meloxicam is under development. No material for the manufacture of the vaginal ring is suggested nor discussed in this document.

All the vaginal rings described so far release sex steroids that inhibit ovulation, including the two that have arrived at the market: Nuvaring (NV Organon, Oss, The Netherlands) and Progering (Laboratories Silesia, Santiago, Chile). Nuvaring releases etonogestrel and ethinyl estradiol, while Progering releases only progesterone, as it is designed specifically for women who are breastfeeding (refs. en Brache and Faundes, Contraception 82:418-427; 2010).

### MECHANISM OF ACTION IN SEX STEROIDS IN VAGINAL RINGS

The mechanism consists in the inhibition of the pituitary gonadotropins by estrogens and progestins and the increase of viscosity of the cervical mucus by the action of progestins to interfere with the migration of sperm to the upper reproductive tract segments (JG Hardman and LE Limbird Eds, Goodman & Gilman's the Pharmacological Basis of Therapeutics, 9th Ed, 1996;. McGraw-Hill, New York Fraser et al Eds, Estrogens and Progestogens in Clinical Practice, 1998.. Churchill Livingstone, London).

Due to the fundamental role of sex steroids in the regulation of the reproductive cycle, contraceptive methods based on the release of hormones necessarily affect the regularity of menstruation and the menstrual cycle. The administration of estrogen is associated with risk of breast cancer which decreases with the use of progestin (L. Speroff et al. Clinical Gynecologic Endocrinology and Infertility, 4th Ed., 1989; Williams & Wilkins, Baltimore, Maryland).

In addition, sex steroids interfere with the medical examination of the hypothalamic-pituitary-gonadal therefore the vaginal ring must be removed one month before the exam, leaving women with no protection during that period.

These considerations highlight the many advantages that would provide a steroid free contraceptive that release a chemical into the vagina, over currently available vaginal rings on the market.

### THE ANOWLATORY EFFECTS OF MEXOLICAM ADMINISTERED ORALLY.

Oral meloxicam (15 mg.) powers the anovulatory effect of a single dose of levonorgestrel (1.5 mg.) administered one week before ovulation. Preovulatory treatments with unique doses of levonorgestrel and levonorgestrel+meloxicam inhibited the follicular rupture by 50% and 70% of the ovulatory cycles respectively (Masai et al. 2007).

Levonorgestrel is a synthetic analogue of the progesterone that activates and binds the progesterone receptors (I.S. Fraser et al. Eds., Estrogens and Progestins in Clinical Practice, 1998; Churchill Livingstone, London).

Meloxicam is a member of the family Oxican, a type of enolic acid with anti-inflammatory, analgesic and antipyretic properties that is sold for the treatment of rheumatic and other inflammatory conditions under approval by the FDA in USA (G. Hardman and L.E. Limbird Eds., Goodman & Gilmans The Pharmacological Basis of Therapeutics, 9th Ed., 1996).

Meloxicam inhibits the COX-2 enzyme that catalyzes the limiting step in the synthesis of inflammatory mediators known as prostaglandins. Prostaglandins are responsible for the breakdown of the follicle wall prior to the expulsion of the egg during ovulation (refs in Massai et al., Human Reproduction 22(2):434-439, 2007; Jesam et al., Human Reproduction 25(2):368-373, 2010).

JESAM et al. (2010) studied the effects of daily oral doses of meloxicam administered for five days of the last stage of the follicular phase, the pattern of hormonal changes and menstrual cycle length. Meloxicam in a dose of 15 mg. and 30 mg. inhibited ovulation in 50% and 91% of cases, respectively. This was the first report about the ovulation inhibition by meloxicam in clinical trials.

### FUNDAMENTALS FOR THE USE OF MELOXICAM ADMINISTERED INTRAVAGINALLY WITH THE PURPOSE TO INHIBIT OVULATION.

The anovulatory effect of meloxicam administered orally has been previously shown, as well as the absorption of active compounds such as sex steroids from the vaginal lumen to female bloodstream with contraception purposes.

This discovery led us to study the passage of meloxicam from an intravaginal device loaded with the medication into the peripheral circulation.

Meloxicam was detected in the peripheral stream a few hours after insertion of a meloxicam loaded device within the vagina.

This evidence makes it possible to propose the use of meloxicam administered from inside the vagina to inhibit ovulation in women. Because of their pharmacological propertied and biochemical similarities, this use can also be extended to other members of the Oxican family such as piroxicam.

Intravaginal administration of meloxicam or piroxicam through device that release drug over prolonged periods of time may also treat different inflammatory problems

### DIFFERENTIATION

This invention is different from intravaginal rings described so far in that the active substance is not a sex steroid, but meloxicam or piroxicam.

This invention does not interfere with the hormonal profile of the ovarian cycle, the metabolism of lipids or carbohydrates, or coagulation.

Specifically inhibit ovulation.

### EXAMPLES

### A) THE MANUFACTURING OF THE DEVICE

**Intravaginal rings of Vinyl plastics:** the ring is manufactured with acetate ethylene of vinyl (EVA, by its acronym in English), low density polyethylene (LDPE for its acronym in English), copolymer of ethylene and 1-octadecene, or combinations of these polymers, up to 6 cm. of outer diameter, with a tube of 4 to 10 mm. wide. The ring is inserted into the vagina.

The polymer or copolymer is loaded with up to 5% of meloxicam. Both are mixed in solid form and molded at a temperature of about 120° C.

### Example 1. Ring manufacturing with low density polyethylene (LDPE)

A solution of 2 g is prepared of LDPE in 100 mL of xylene under stirring at 120° C. The solution is poured into 300 mL of acetone and then stirred for 2 minutes, filtered under vacuum. The resulting solid polymer pieces are dried at 37 ° C. The fragments are sprayed by hand until the solvents are completely evaporated. The polymer is mixed with meloxicam and molded at 120° C for 1 hour

### Example 2. Manufacturing meloxicam loaded rings made of low density polyethylene (LDPE)

Meloxicam is dried at 140 ° C for 30 minutes. LDPE is mixed with meloxicam at room temperature and is molded at 120 ° C for approximately 15 minutes. The mixture is compressed manually and is molded at 120 º for 40 minutes. The proportion of meloxicam is set from 1% to 5% of the substance in the LDPE.

### Example 3: Manufacturing a ring loaded with meloxicam made with EVA (ethylene vinyl acetate).

EVA is dissolved in xylene at 130 ° C stirring. After cooling for 5 minutes at room temperature the solution of excess acetone (1:50) is poured. After filtration, the EVA is dried at 37 ° C. The meloxicam and EVA will be completely mixed (1% to 5% meloxicam) and molded at 120 ° C for 50 minutes.

### Example 4. Manufacturing a ring of copolymer of ethylene and 1-octadecene with meloxicam and piroxicam

A copolymer of ethylene and and 1-octadecene is prepared at 1 atm of ethylene gas with 5 mL of 1-octadecene shaking at 500 rpm (60 °) in an oxygen-free environment (chamber with N2 gas).

The copolymer of ethylene and 1-octadecene (1.54 x 10-2 mol 1-octadecene) was then dissolved in xylene at 130 ° C. After cooling for 20 minutes at room temperature, the solution is poured excess acetone, and the precipitate is filtered and dried under vacuum for 30 minutes. For a 1% meloxicam ring copolymer of ethylene and 1-octadecene, meloxicam is dried at 140 ° C for 30 minutes and thoroughly mixed with the copolymer. This blend is molded for 15 minutes at 120 ° C before applying further pressure and mold for another 40 minutes. The proportion of meloxicam can be increased by about 5% in poly 1-octadecene.

To prepare a piroxicam ring to 1%, the drug is first dried at 130 ° C for 45 minutes. After cooling for 10 minutes at room temperature, the piroxicam is mixed with the copolymer by mechanical stirring for 50 minutes. The resulting mixture is molded at 130 ° C for 5 minutes before being compressed and molded more time for another 30 minutes. The piroxicam concentration can be increased to approximately 5%.

### Example 5. Manufacturing of copolymer LDPE: EVA (50:50) with meloxicam

A solution of LDPE and EVA (50:50) in xylene (1:50 weight: vol) is prepared at 130 ° C. After cooling for 2 to 3 minutes at room temperature, the solution of acetone (1:50) is poured to induce precipitation of the copolymer. The solution is vacuum filtered and the precipitate is dried at 37 ° C for 2 hours.

### B) The release of meloxicam and piroxicam from polymeric devices.

### Example 6. Meloxicam release from rings of loaded LDPE with 1% or 3% of meloxicam.

The results demonstrate that meloxicam is diluted from the polymer matrix into the aqueous incubation medium (phosphate buffer, pH 7.4, 37 ° C) at a constant rate from the second day.

**Liberation of meloxicam (µg/24 h):**

| Sample | % Meloxicam | Day 1 | Day 2 | Dia 4 | Dia 8 |
|---|---|---|---|---|---|
| A | 1 | 70,3 | 5,2 | 2,3 | 2,9 |
| B | 1 | 64,7 | 4,1 | 1,8 | 2,3 |
| C | 3 | 32,6 | 2,8 | 2,1 | 2,1 |
| D | 3 | 36,2 | 3,3 | 1,6 | 1,2 |

**Rate of release of meloxicam according to weight (µg/µg LDPE / 24 h)**

| Sample | % Meloxicam | Day 1 | Day 2 | Day 4 | Day 8 |
|---|---|---|---|---|---|
| A | 1 | 513 | 37,9 | 16,8 | 21,1 |
| B | 1 | 425 | 26,7 | 11,8 | 15,1 |
| C | 3 | 281 | 23,8 | 18,1 | 18,1 |
| D | 3 | 310 | 28,6 | 13,7 | 10,3 |

### Example 7. Meloxicam release from a ring of EVA loaded with a 1% of meloxicam.

**Incubation in 6.5 ml of phosphate buffer pH 7.4 at 37 ° C.**

| Day of Incubation | Meloxicam (µg/mL) | Total Release (µg) |
|---|---|---|
| 1 | 282,0 | 18330 |
| 3 | 59,1 | 1921 |
| 13 | 55,9 | 363 |
| 23 | 41,8 | 272 |
| 30 | 38,6 | 251 |

The results demonstrate that meloxicam is released from the EVA matrix to the saline aqueous medium during the 30 day incubation period.

### Example 8. Levels of meloxicam in the bloodstream after insertion of the meloxicam intravaginal ring.

The drug was measured in 3 women (average age 21 years) after insertion of a ring loaded with 5% meloxicam in the vagina. Meloxicam level using a high resolution liquid chromatography was measured. These results demonstrate that meloxicam is diluted from the vaginal lumen into peripheral circulation

**Elapsed time after Meloxicam [microgram/mL]**

| **insertion** | |
|---|---|
| 0 h | Not-detectable |
| 3 h | 1,8 ± 0,5 (mean ± standard error) |

### Example 9.

A polymer EVA-LPE ring with 1% of piroxicam is manufactured. Separately incubated ring segments of two hundred mg. in 7 mL of phosphate buffer (pH 7.4, 37 ° C). The level of piroxicam was measured in an incubation medium using a high-resolution liquid chromatography. At 48 hours of incubation they were 56.3 ± 2.5 µg/mL of piroxicam or in the incubation medium (n = 10) (mean ± standard error).

These results show that the piroxicam is released into an aqueous saline medium from the copolymer matrix.

### Example 10:

The meloxicam is released from a manufactured copolymer of ethylene and 1-octadecene ring. Total release per 24 hours is reported.

| Time of Incubation | µg/24 h | |
|---|---|---|
| 48 h | 61,5 ± 7,0 | (n=3) |
| 96 h | 9,5 ± 2,0 | (n=3) |
| 168 h | 17,0 ± 2,2 | (n=3) |

These results demonstrate that meloxicam is diluted from the matrix 1-octadecene to the incubation medium (phosphate buffer, pH 7.4, 37 ° C). The concentration in the medium is also reported (ug / mL).

| Time of Incubation | Medium | Standard Error | n |
|---|---|---|---|
| 96 h | 36,8 | 4,8 | 4 |
| 144 h | 5,7 | 0,9 | 4 |

The release rate of meloxicam from the EVA-LDPE copolymer is similar to the rates obtained from the rings manufactured with EVA or with unblended LDPE. This similarity would allow flexibility to modify the ring varying the proportions of the two plastics, which, in turn, may allow for adjustment of the parameters to the particular needs of different users.

## Claims

1. An intravaginal ring to inhibit women ovulation **CHARACTERIZED in that** the ring is loaded with meloxicam or piroxicam and that is made of vinyl polymers.

2. The intravaginal ring as claimed in claim 1, **CHARACTERIZED in that** said vinyl polymer is loaded with up to 5% meloxicam or piroxicam.

3. The intravaginal ring as claimed in claim 1, **CHARACTERIZED in that** said vinyl polymer is low density polyethylene (LDPE).

4. The intravaginal ring as claimed in claim 1, **CHARACTERIZED in that** said vinyl polymer is an ethylene and 1-octadecene copolymer.

5. The intravaginal ring as claimed in claim 1, **CHARACTERIZED in that** said vinyl polymer is an ethylene-vinyl acetate polymer (EVA).

6. The intravaginal ring as claimed in claim 1, **CHARACTERIZED in that** said ring is made from EVA and LDPE at a ratio between 0.1-0.9:0.9-0.1.

7. The intravaginal ring as claimed in claim 1, **CHARACTERIZED in that** said ring is made from EVA and LDPE at a ratio of 0,5:0,5.

## Patentansprüche

1. Intravaginalring zur Hemmung des Eisprungs bei Frauen, **dadurch gekennzeichnet, dass** der Ring mit Meloxicam oder Piroxicam beladen ist und aus Vinylpolymeren hergestellt ist.

2. Intravaginalring nach Anspruch 1, **dadurch gekennzeichnet, dass** das Vinylpolymer mit bis zu 5 % Meloxicam oder Piroxicam beladen ist.

3. Intravaginalring nach Anspruch 1, **dadurch gekennzeichnet, dass** das Vinylpolymer Polyethylen niedriger Dichte (LDPE, engl. low density polyethylene) ist.

4. Intravaginalring nach Anspruch 1, **dadurch gekennzeichnet, dass** das Vinylpolymer ein Ethylen und 1-Octadecen-Copolymer ist.

5. Intravaginalring nach Anspruch 1, **dadurch gekennzeichnet, dass** das Vinylpolymer ein Ethylenvinylacetatpolymer (EVA) ist.

6. Intravaginalring nach Anspruch 1, **dadurch gekennzeichnet, dass** der Ring aus EVA und LDPE in einem Verhältnis zwischen 0,1-0,9 : 0,9-0,1 hergestellt ist.

7. Intravaginalring nach Anspruch 1, **dadurch gekennzeichnet, dass** der Ring aus EVA und LDPE in einem Verhältnis von 0,5 : 0,5 hergestellt ist.

## Revendications

1. Anneau intravaginal pour inhiber l'ovulation chez la femme **CARACTÉRISÉ en ce que** l'anneau est chargé en méloxicam ou piroxicam et qu'il est fait de polymères de vinyle.

2. Anneau intravaginal tel que revendiqué dans la revendication 1, **CARACTÉRISÉ en ce que** ledit polymère de vinyle est chargé jusqu'à 5 % en méloxicam ou piroxicam.

3. Anneau intravaginal tel que revendiqué dans la revendication 1, **CARACTÉRISÉ en ce que** ledit polymère de vinyle est du polyéthylène basse densité (LDPE).

4. Anneau intravaginal tel que revendiqué dans la revendication 1, **CARACTÉRISÉ en ce que** ledit polymère de vinyle est un copolymère d'éthylène et d'1-octadécène.

5. Anneau intravaginal tel que revendiqué dans la revendication 1, **CARACTÉRISÉ en ce que** ledit polymère de vinyle est un polymère éthylène-acétate de vinyle (EVA).

6. Anneau intravaginal tel que revendiqué dans la revendication 1, **CARACTÉRISÉ en ce que** ledit anneau est fait à partir d'EVA et de LDPE dans un rapport compris entre 0,1-0,9:0,9-0,1.

7. Anneau intravaginal tel que revendiqué dans la revendication 1, **CARACTÉRISÉ en ce que** ledit anneau est fait à partir d'EVA et de LDPE dans un rapport de 0,5:0,5.
